# EUROPEAN PATENT APPLICATION

(11) **EP 0 962 584 A1**
(43) Date of publication of application: **08.12.1999**
(21) Application number: 99104555.0
(22) Date of filing: 08.03.1999
(51) Int. Cl.: D06M 23/08, D06M 13/422, D06M 11/00, D06M 13/00

(54) **Fabric having deodorizing function and method of deodorizing fabric**

(30) Priority: 02.06.1998 JP 15271998; 08.09.1998 JP 25409698
(71) Applicant: Suminoe Textile Co., Ltd., Osaka (JP)
(72) Inventor: Seto, Yasutaro, Yao-shi, Osaka (JP); Gennaka, Shuichi, Kitakatsuragi-gun, Nara (JP)
(74) Representative: Grünecker, Kinkeldey, Stockmair & Schwanhäusser Anwaltssozietät

(57) **Abstract**

A fabric which is good in drape, high in removability, and can maintain removal ability for a long time even after washing, and a method of deodorizing which can produce such a fabric efficiently at a low cost without limiting the kind of material yarn. A deodorant comprising an amine compound having an average particle diameter of 10 µm or less is fixed to yarn forming a fabric by a binder resin having a glass transition point of -30 °C or lower. Preferably, an inorganic substance is further used as an additional deodorant. In the deodorizing method, the fabric is treated with a treating agent containing the above specific deodorant and binder resin to bond it to the yarn forming the fabric.

## Description

### BACKGROUND OF THE INVENTION

This invention relates to a fabric such as a carpet having a deodorizing function, i.e. the function of efficiently removing airborne chemical and other ill-smelling substances such as formaldehyde, ammonium and acetic acid.

People are becoming increasingly conscious of foul odors in their living environments. But since modern houses are highly airtight, it is difficult to remove foul smells. Foul smells in living environments originate from e.g. ammonium, trimethylamine, hydrogen sulfide, methyl mercaptan, acetic acid, and most notoriously, cigarette smoke.

On the other hand, chemical substances such as formaldehyde, which come from floor and wall materials, reportedly, stimulate human eyes, noses and skin, causing various complaints even if its concentration is so low as not to be recognized by humans. Such a substance has an especially serious influence on people who are sensitive to chemical substances. In fact, for those people, it is difficult even to lead a normal life. This disease, known as "sick house syndrome", has become one of the modern social problems.

In living environments, besides those substances that produce foul smells, there are chemical substances that may cause the sick house syndrome even if their concentration is so low as not to be recognizable by humans, such as formaldehyde.

Carpets that can remove foul smells are now available. Since carpets have a relatively large area, they can contact air over a large area, so that they can remove smells efficiently.

Ordinary such deodorizing carpets are made from piled yarn having deodorizing properties. Such deodorizing yarn is manufactured by spinning a resin material in which is uniformly kneaded a deodorant.

Deodorizing yarns for commercially available deodorizing carpets are almost all made from polyester or acrylic resins, and thus less variable in yarn number. There are practically no commercial deodorizing carpets made from union yarns. To answer customers' requirements, piled yarn made from a different material is sometimes used at a specific portion of a carpet. Thus in manufacturing a deodorizing carpet, it was often necessary to use a normal, non-deodorizing yarn at a specific portion of the carpet. In short, the range of choice in the kind of resin material for the yarn, yarn number and gloss is limited.

Properties required for carpets besides deodorizing properties include feeling to the touch, flexibility, resilience, elastic recovery, walkability, water resistance, flame retardancy, chemical resistance, wear resistance, sound insulating properties, heat insulating properties, antistatic properties, and many more. Material design including the selection of the material for pile yarn and that of pile number is made according to the intended use. But the range of such material design was extremely limited, so that it was applicable only to very limited use. Thus, conventional deodorizing carpets were not satisfactory in flexibility of use.

Further conventional deodorizing yarns were relatively expensive and thus carpets made from such yarns were also expensive.

Also, carpets fabricated from conventional such deodorizing yarns were rather short in the duration of deodorizing effects.

Besides carpets, there are also curtains, cloths, moquettes for chair lining made of deodorizing yarns. But like deodorizing carpets, they are inferior in flexibility of use, expensive, insufficient in the deodorizing effects, and less lasting in deodorizing effect. Thus, only very few deodorizing fabrics are now commercially avaible.

An object of this invention is to provide a deodorizing fabric such as a carpet which is elegant in appearance and to the touch, and can remove smells efficiently for a long time even after laundering.

Another object is to provide a method of efficiently manufacturing such fabrics out of many different kinds of yarns at a low cost.

### SUMMARY OF THE INVENTION

The deodorizing fabric according to this invention includes a deodorant comprising an amine compound having an average particle diameter of 10 µm or less and adhered to the yarn forming the fabric by means of a binder resin having a glass transition point of -30 °C or lower.

The amine compound adhered to the fabric-forming yarn removes chemical or odor substances such as formaldehyde and acetoaldehyde. Since the amine compound has an average particle diameter of 10 µm or under, it will not give the fabric a sandy feeling to the touch. It gives good drape too. Since the binder resin has a glass transition point of -30°C or lower, it will not harden the fabric. Thus, elegant drape is maintained. Further, the binder resin securely adheres to the yarn, preventing the deodorant from coming off the fabric, so that the deodorizing effect lasts long.

Another deodorizing fabric of this invention has a deodorant comprising an amine compound having an average particle diameter of 10 µm or less, and an inorganic substance and adhered to the yarn forming the fabric by means of a binder resin having a glass transition point of -30 °C or lower.

The amine compound adhered to the fabric-forming yarn removes chemical or odor substances such as formaldehyde and acetoaldehyde. The inorganic substance contained in the deodorant removes chemical or odor substances that are not sufficiently removed by the amine compound, such as ammonium and acetic acid. Thus, the amine compound and the inorganic substance cooperate with each other to remove almost all of main chemical and odor substances in a room.

Since the amine compound has an average particle diameter of 10 µm or under, it will not give the fabric a sandy feeling to the touch. Drape is good too. Since the binder resin has a glass transition point of -30°C or lower, it will not harden the fabric. Thus, elegant drape is maintained. Further, the binder resin securely adheres to the yarn, preventing the deodorant from coming off the fabric, so that the deodorizing effect lasts long.

In the above, the amine compound or compounds are preferably one or two kinds of compounds selected from the group consisting of hydrazine derivatives having a water solubility of 5 g/L or less at 25 °C.

By using a hydrazine derivative or derivatives as the amine compound, the odor-removing properties improve. Since their water solubility is 5 g/L or under at 25 °C, the hydrazine derivative or derivatives will never dissolve into water even if brought into contact with water in laundering.

In order to prevent the deodorant from coming off the fabric and thus to maintain the deodorizing function for a long tine and to maintain good drape, the deodorant-to-binder resin weight ratio is preferably between 10/50 and 10/2.

The deodorant is preferably present at the rate of 5-30 g/m² if the balance of cost and deodorizing function is taken into account.

The fabric deodorizing method according to this invention comprises the step of treating a fabric with a treating agent containing a deodorizing composition comprising a deodorant comprising an amine compound having an average particle diameter 10 µm or less, and a binder resin having a glass transition point of -30 °C or lower to adhere the deodorizing composition to the yarn forming the fabric.

Another fabric deodorizing method according to this invention comprises the step of treating a fabric with a treating agent containing a deodorizing composition comprising a deodorant comprising an amine compound having an average particle diameter 10 µm or less, and an inorganic substance, and a binder resin having a glass transition point of -30 °C or lower to adhere the deodorizing composition to the yarn forming the fabric.

With either method, since the treating agent contains a specific deodorant having a specific particle diameter and a specific binder, the fabric formed has elegant drape, and high and long-lasting deodorizing properties. In either method, since the deodorizing treatment is applied to yarn that has been formed into a fabric, that is, applied to a fabric to which material design has been made for various elements such as selection of the type of yarn, these methods are applicable to any use or any form of fabric. Basically, in these methods, deodorizing properties are given to the fabric by subjecting the fabric to deodorizing treatment such as spraying and immersing only once. Thus, deodorizing fabrics are manufacturable efficiently at a low cost.

Preferable deodorizing treatment is spraying because spraying improves productivity.

Other features and objects of the present invention will become apparent from the following description made with reference to the accompanying drawing, in which:

### BRIEF DESCRIPTION OF THE DRAWINGS

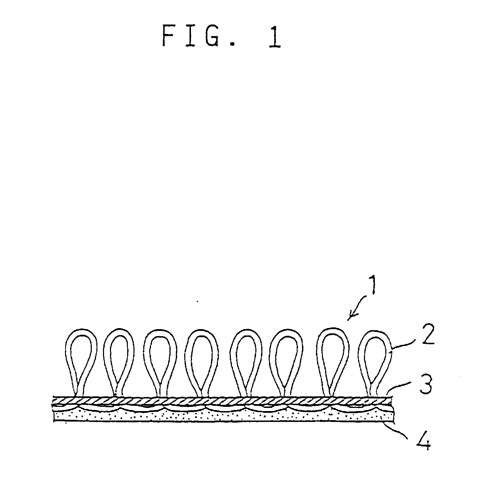

Fig. 1 is a sectional view of a fabric embodying this invention.

### DETAILED DESCRIPTION OF THE PREFERRED EMBODIMENT

The fabric 1 of this embodiment is a carpet comprising a substrate 3 having a pile layer 2 and a lining 4 laminated on the back of the substrate 3. The pile layer 2 carries a deodorant.

The deodorant adhered to the yarn forming the fabric 1 is described first. As the deodorant, an amine compound is used. The amine compound is not limited but preferably a hydrazine derivative. Such an amine compound can adsorb chemical substances such as formaldehyde, acetoaldehyde and acetic acid. The amine compound has preferably a water solubility of 5 g/L or less at 25 °C. If the water-solubility of the amine compound is in such a low range, the less it is likely to dissolve into water when brought into contact with water in e.g. laundering. This enable the amine compound to exhibit its deodorizing properties for a prolonged period of time.

Usable hydrazine derivatives include those obtained by reacting a hydrazine compound with a long-chain aliphatic compound, and those obtained by reacting a hydrazine compound with an aromatic compound.

More specifically, the following are more preferable: reaction products of one or two kinds of compounds selected from the group consisting of hydrazine and semicarbazide with one or more kinds of compounds selected from the group consisting of monocarboxylic acid, dicarboxylic acid, aromatic monocarboxylic acid and aromatic dicarboxylic acid all having 8-16 carbon atoms; and reaction products of one or two kinds of compounds selected from the group consisting of hydrazine and semicarbazide with one or more kinds of compounds selected from the group consisting of a monoglycidyl derivative and a diglycidyl derivative both having 8-16 carbon atoms. The use of such hydrazine derivatives further improve deodorizing properties. Specifically, such reaction products include, but are not limited to, dihydrazide sebacate, dihydrazide dodecanoic diacid and dihydrazide isophthalate.

In this invention, it is preferable to use an inorganic substance as a second deodorant. Usable inorganic substances include, but are not limited to, active carbon, zeolite, montmorillonite, silica gel, and metallic oxides such as alumina, titanium oxide, zinc oxide and iron oxide. In cooperation with the amine compound, such an inorganic substance removes main chemical substances present in the room.

The inorganic substance should preferably be a porous one because a porous substance is larger in surface area and thus can adsorb a greater amount of substances. Also, by using a porous inorganic substance, the amine compound will enter into the pores of the porous substances, thus more effectively adsorbing and decomposing chemical substances in cooperation with the inorganic substance. Such porous inorganic substances include activated carbon and zeolite. Zeolite is particularly preferable because it is high in the ability to adsorb acetic acid and ammonium. In cooperation with the amine compound, zeolite can remove main chemical substances in the room.

According to this invention, the amine compound and the inorganic substance used as deodorants have to have an average particle diameter not exceeding 10 µm. Over 10 µm, they will give the fabric a rough feeling to the touch. Drape will worsen too. Their average particle diameter is preferably 5 µm or less, and more preferably 3 µm or less.

On the other hand, the binder resin has to have a glass transition point (Tg) of -30°C or lower. Over -30°C, the drape of the fabric will deteriorate markedly. Its glass transition point is preferably -35°C or lower.

The binder resin may be any resin having a glass transition point (Tg) of -30 °C or lower, such as self-crosslinking acryl resin, metacrylic resin, urethane resin, silicone resin, glyoxal resin, vinyl acetate, vinylidene chloride resin, butadiene resin, melamine resin, epoxy resin, acrylic-silicone copolymer resins, ethylene-vinyl acetate copolymer resins, isobuthylene-anhydrous maleic acid copolymer resins, ethylene-styrene-acrylate-metacrylate copolymer resins, or a mixture thereof.

According to this invention, the weight ratio of the deodorant to the binder resin is preferably 10/50 to 10/2. If this ratio is higher than this range, the deodorant can easily come off the fabric, thus shortening the durability of the deodorizing effect. On the other hand, if this ratio is below the above range, drape of the fabric will deteriorate, and the deodorizing properties will worsen. The deodorant-to-binder resin weight ratio is more preferably between 10/20 and 10/2.

The deodorant should preferably be on the fabric at the rate of 5-30 g/m² (in dried state). Under 5 g/m², its deodorizing ability will be insufficient. Over 30 g/m², no further improvement in its deodorizing ability is expectable. Only the cost increases.

For the carpet of this embodiment, the carpet substrate may be of any kind. For example, it may be made of synthetic fiber such as polyester fiber, nylon fiber, polypropylene fiber or acrylic fiber, or may be a fabric made by weaving or knitting yarn made of natural fiber such as hemp, cotton or wool, or a nonwoven fabric formed by mechanically binding together various kinds of fibers or yarns by needling or by chemically binding together fibers or yarns with an adhesive.

The pile material of the pile layer is not limited and may be polyester fiber, nylon fiber, polypropylene fiber, acrylic fiber, rayon fiber, or natural fiber such as hemp, cotton or wool. The method of forming the pile layer is not limited, either. The pile layer may be formed by weaving such as warp or weft pile weaving, by planting pile yarn with a tufting machine, by a weaving machine, or by bonding pile yarn with an adhesive. The piles may be cut piles, loop piles or of any other type.

The material of the lining layer is not limited and may be a resin composition, a rubber composition, jute, polypropylene fabric, and needle punch fabric. Ordinarily, a resin or rubber composition is used. The resin component of the resin composition may be acrylic, urethane, polyvinyl chloride, polyethylene, polypropylene or ethylene-vinyl acetate copolymer (EVA) resin. The rubber component of the rubber composition may be SBR (styrene-butadiene rubber), MBR (methyl methacrylate-butadiene rubber), NBR (acrylonitrile butadiene rubber), or natural rubber.

In this invention, the fabric may be of any kind. Besides a carpet, it may be curtains, roll screens, cloths, moquettes for chair lining, furniture cloth, drop curtains, tapestry, photographic cloth, wrapping cloth, table centers and art cloth. Yarns for these fabrics may not be limited, but include polyester fibers, nylon fibers, polypropylene fibers, acrylic fibers, rayon fibers and natural fibers such as hemp, cotton and wool.

The deodorizing fabric according to this invention may be manufactured in e.g. the following manner. A treating agent comprising an aqueous dispersion in which a deodorant and a binder resin are dispersed in water is prepared. The deodorant and binder resin should be dispersed as uniformly as possible. For the binder resin, it is preferable to cause it to form an emulsion state with water. Alcohol may be used as a dispersion medium instead of water. But water is preferable.

In preparing the dispersion, the deodorant is preferably dispersed in water first, and then a binder resin is dispersed. This order is preferable to disperse the deodorant and the binder resin more uniformly.

To improve the properties of treating agents, various additives such as a dispersant and a thickener may be added to the treating agents. Further, in order to improve various properties of the fabric, additives such as antibacterial agents, hydrophilicity giver and flame-retardants may be added to such an extent that the advantage of the invention will not lessen.

The fabric is treated with such a treating agent to apply the deodorizing composition to the fabric-forming yarn. Means for applying is not limited and may be spraying, foaming, immersing, coating or padding. Among them, spraying is most preferable in view of workability, production efficiency and deodorizing cost.

After deodorizing treatment, the fabric is dried. Drying means is not limited, and may be by air or by heat treatment. Considering the efficiency of drying, heat treatment is preferable.

The temperature for heat treatment is preferably 100-180°C. By performing heat treatment within this temperature range, the deodorizing composition can be adhered securely to the fabric. This prolongs the duration of the deodorizing effect.

The fabric according to this invention is not limited to one manufactured by the abovesaid deodorizing method but may be made e.g. by adhering a deodorant to yarn which has not yet been formed into a fabric by means of a binder resin having a glass transition point of - 30°C or lower, and then forming the yarn into a fabric.

### [Examples]

### 〈Materials used〉

### (fabric A) carpet

- carpet substrate :: fabric of polypropylene fiber
- pile layer :: made on the substrate by tufting pile yarn made of polyester fiber (weight per unit area 700 g/m²)
- lining layer :: SBR latex

### (fabric B) carpet

- carpet substrate :: fabric of polypropylene fiber
- pile layer :: made on the substrate by tufting pile yarn made of acrylic fiber (600 g/m²)
- lining layer :: SBR latex

### (fabric C) carpet

- carpet substrate :: fabric of polypropylene fiber
- pile layer :: made on the substrate by tufting pile yarn made of ester filament (700 g/m²)
- lining layer :: ethylene-vinyl acetate copolymer (EVA)

### (fabric D) moquette

- substrate :: fabric of polypropylene fiber
- pile layer :: formed on the substrate by weaving pile yarn made of polyester fiber
- lining layer :: acrylic latex

### (fabric E) curtain

fabric of polypropylene fiber (250 g/m²)

### (fabric F) cloth

fabric of rayon fiber (300 g/m²)

### 〈Example 1〉

A dispersion was prepared by adding 4 parts by weight of dihydrazide sebacate having an average particle diameter of 4 µm to 92 parts by weight of water, and agitating the mixture. To this dispersion, 4 parts by weight of acrylic resin (binder resin having Tg of -37°C) was added and the mixture was agitated sufficiently to prepare a uniform dispersion (which is a treating agent). A deodorizing carpet was prepared by applying the treating agent to a fabric by spraying, and drying the fabric for 10 minutes at 130 °C.

### 〈Examples 2-4〉

Deodorizing carpets were prepared in exactly the same way as in Example 1 except that treatment was carried out using the treating agents shown in Table 1 under the conditions shown in Table 1.

### 〈Example 5〉

A deodorizing moquette was prepared in exactly the same way as in Example 1 except that treatment was performed using the treating agent shown in Table 2 under the conditions shown in Table 1.

### 〈Example 6〉

A deodorizing curtain was prepared in exactly the same way as in Example 1 except that treatment was performed using the treating agent shown in Table 2 under the conditions shown in Table 1 by padding.

### 〈Example 7〉

A deodorizing cloth was prepared in exactly the same way as in Example 1 except that treatment was performed using the treating agent shown in Table 2 under the conditions shown in Table 1 by padding.

### 〈Comparative Example 1〉

A deodorizing carpet was prepared in the same way as in Example 1 except that instead of dihydrazide sebacate having an average particle diameter of 4 µm, dihydrazide sebacate having an average particle diameter of 12 µm was used.

### 〈Comparative Example 2〉

A deodorizing carpet was prepared in the same way as in Example 1 except that instead of an acrylic resin having a Tg of -37°C, an acrylic resin having a Tg of -20 °C was used.

The following tests A-D were conducted for the thus formed fabrics. The results are shown in Table 3.

### 〈Tests and Evaluation〉

### A. Removability test

### (Formaldehyde removability)

A test piece (10 x 10 cm square) cut from each fabric was put in a 3-liter bag, and formaldehyde gas was introduced into the bag until its concentration rises to 200 ppm. The concentration of the remaining formaldehyde was measured 48 hours after the introduction of the gas. Based on the measured value, the total amount of gas adsorbed and removed by each fabric specimen was calculated, and based on this value, the formaldehyde gas removal rate (%) was calculated.

### (Ammonium removability)

The ammonium gas removal rate (%) for each specimen was calculated in the same way as the above test except that instead of formaldehyde gas, ammonium gas was introduced into the bag until its concentration rises to 200 ppm.

### (Acetate acid removal ability)

The acetate acid gas removal rate (%) for each specimen was calculated in the same way as the above test except that instead of formaldehyde gas, acetic acid gas was introduced into the bag until its concentration rises to 200 ppm.

### B. Test for the durability of the removability.

Each fabric was washed three times and after natural drying, subjected to the same removability test as above to calculate the drop rate (%) of the removability for each of the above three kinds of gases. The average of the drop rates for the respective gases were then calculated.

Removability drop rate (%) = (total amount of gas adsorbed after laundering) ÷ (total amount of gas adsorbed before laundering) x 100

### C. Drape Evaluation

The difference in drape (or hardness) of each fabric before and after deodorizing treatment was evaluated by directly touching it by hand.

### (Standard of evaluation)

- ○: difference was scarcely felt
- △: difference was slightly felt
- X: significant difference was felt

### D. Feeling (roughness) to the touch

The feeling to the touch was evaluated.

### (Standard of evaluation)

- ⓞ: no rough feeling
- ○: little rough feeling
- △: slight rough feeling
- X: marked rough feeling

### 〈Results of evaluation〉

As will be apparent from Table 3, the fabrics of Examples 1-7 according to this invention were smooth to the tough, high in drape and the ability to remove formaldehyde, and maintained this ability for a long period of time.

In contrast, the fabric of Comparative Example 1, which is out of the scope of this invention, was rough to the touch and poor in drape. The fabric of Comparative Example 2 was inferior in drape.

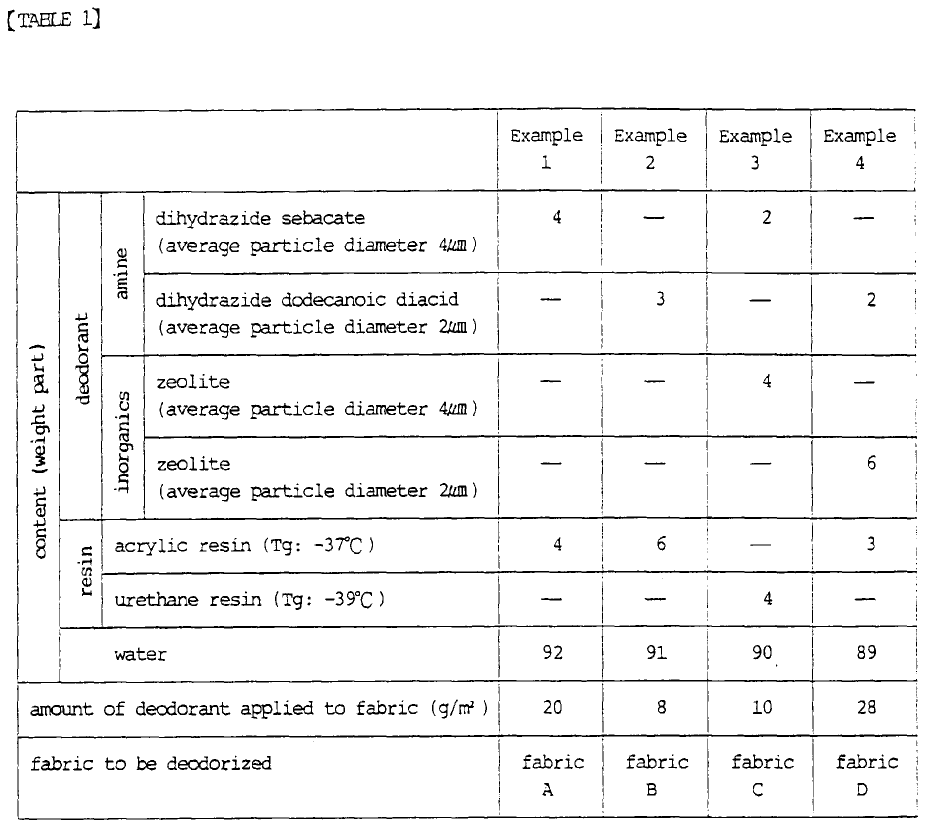

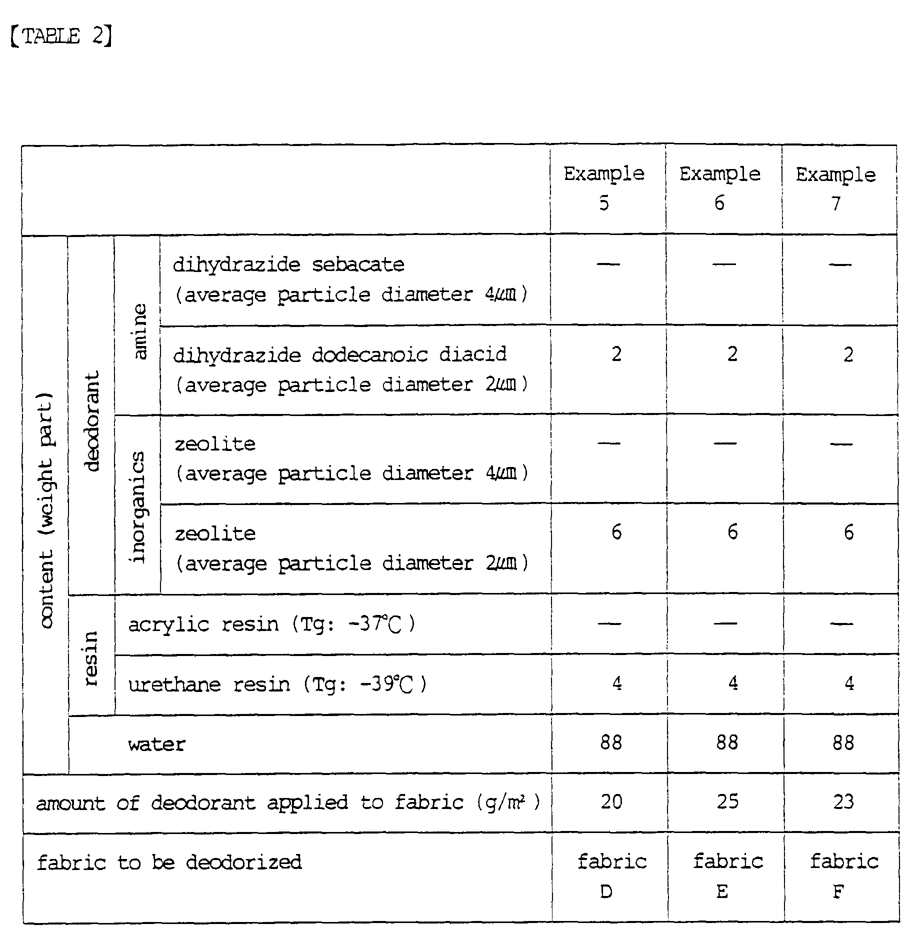

**[TABLE 3]**

| | removability | | | drop rate of removability (%) | drape | roughness |
|---|---|---|---|---|---|---|
| | formaldehyde removability (%) | ammonium removability (%) | acetic acid removability (%) | | | |
| Example 1 | 80 | 20 | 33 | 92 | ○ | ○ |
| Example 2 | 70 | 13 | 25 | 88 | ○ | ○ |
| Example 3 | 62 | 67 | 70 | 90 | ○ | ○ |
| Example 4 | 83 | 70 | 75 | 95 | ○ | ⓞ |
| Compara. Example 1 | 76 | 20 | 31 | 90 | ○ | X |
| Compara. Example 2 | 72 | 25 | 30 | 91 | X | ⓞ |
| Example 5 | 80 | 75 | 80 | 93 | ○ | ⓞ |
| Example 6 | 87 | 80 | 88 | 95 | ○ | ⓞ |
| Example 7 | 82 | 78 | 86 | 94 | ○ | ⓞ |

## Claims

1. A fabric having a deodorizing function wherein a deodorant comprising an amine compound having an average particle diameter of 10 µm or less is adhered to a yarn forming said fabric by means of a binder resin having a glass transition point of -30 °C or lower.

2. A fabric having a deodorizing function wherein a deodorant comprising an amine compound having an average particle diameter of 10 µm or less and an inorganic substance is adhered to a yarn forming said fabric by means of a binder resin having a glass transition point of -30°C or lower.

3. The fabric of claim 1 or 2 wherein said amine compound is one or more than one compound selected from the group consisting of hydrazine derivatives having a water solubility of 5 g/L or less at 25°C.

4. The fabric of any of claims 1-3 wherein the weight ratio of said deodorant to said binder resin is 10/50 to 10/2.

5. The fabric of any of claims 1-4 wherein said deodorant is present at the rate of 5-30 g/m².

6. A method of subjecting a fabric to deodorizing treatment by treating the fabric with a treating agent containing a deodorizing composition comprising a deodorant comprising an amine compound having an average particle diameter of 10 µm or less and a binder resin having a glass transition point of -30°C or lower, whereby adhering said deodorizing composition to yarn forming the fabric.

7. A method of subjecting a fabric to deodorizing treatment by treating the fabric with a treating agent containing a deodorizing composition comprising a deodorant comprising an amine compound having an average particle diameter of 10 µm or less and an inorganic substance and a binder resin having a glass transition point not exceeding -30°C or lower, whereby adhering said deodorizing composition to yarn forming the fabric.

8. The method of claim 6 or 7 wherein the treatment of said fabric by said treating agent is performed by spraying.
